# EUROPEAN PATENT SPECIFICATION

(11) **EP 0 783 475 B1**
(45) Date of publication and mention of the grant of the patent: **09.12.1998**
(21) Application number: 95934652.9
(22) Date of filing: 28.09.1995
(51) Int. Cl.: C07C 45/58, C07C 47/19, C07C 31/20, C07C 29/141, C07C 45/80

(54) **PROCESS FOR PREPARING 1,3-ALKANEDIOLS AND 3-HYDROXYALDEHYDES**
VERFAHREN ZUR HERSTELLUNG VON 1,3-ALKANDIOLEN UND 3-HYDROXYALDEHYDEN
PROCEDE DE PREPARATION DE 1,3-ALCANEDIOLS ET DE 3-HYDROXYALDEHYDES

(30) Priority: 30.09.1994 US 316673; 30.09.1994 US 316661
(43) Date of publication of application: 16.07.1997
(73) Proprietor: SHELL INTERNATIONALE RESEARCH MAATSCHAPPIJ B.V., 2596 HR Den Haag (NL)
(72) Inventor: ARHANCET, Juan, Pedro, Katy, TX 77450 (US); FORSCHNER, Thomas, Clayton, Richmond, TX 77469 (US); POWELL, Joseph, Broun, Houston, TX 77070 (US); SEMPLE, Thomas, Carl, Friendswood, TX 77546 (US); SLAUGH, Lynn, Henry, Houston, TX 77070 (US); THOMASON, Terry, Blane, Houston, TX 77077 (US); WEIDER, Paul, Richard, Houston, TX 77083 (US)
(86) International application number: EP9503868
(87) International publication number: WO9610550

(56) References cited:
- WO-A-94/18149
- US-A- 3 687 981
- US-A- 4 973 741

## Description

This invention relates to a process for preparing 1,3-alkanediols and 3-hydroxyaldehydes by hydroformylating an oxirane (1,2-epoxide). In particular, the invention relates to a cobalt-catalysed process for preparing 1,3-propanediol by hydroformylating ethylene oxide and hydrogenating the hydroformylation product.

The hydroformylation of oxiranes to produce 3-hydroxyaldehydes has been discussed in "New Syntheses with Carbon Monoxide" edited by J. Falbe (1980), pp. 131-132. The reaction is catalysed with a cobalt-based catalyst, or a phosphine-modified cobalt-based catalyst. The hydroformylation product, be it the 3-hydroxyaldehyde or the cyclic hemiacetal that is its dimer, may be converted into a 1,3-alkanediol by hydrogenation. This process is of particular importance to the preparation of 1,3-propanediol (PDO), an intermediate in the production of polyesters for fibres and films, that may be prepared by hydrogenating 3-hydroxypropanal (HPA). According to this textbook attempts have been made to improve the hydroformylation reaction by adding small amounts of alcohols, ethers, ketones and esters. According to US-A-3 687 981 also halogen containing inorganic compounds, e.g., inorganic salts and acids such as hydrochloric acid may be used as hydroformylation promoter.

Finally, from WO94/18149 a process for preparing 1,3-alkanediols and 3-hydroxyaldehydes is known, which process is carried out in the presence of a phosphine-ligated cobalt catalyst. This process leaves room for improvement in respect of the reaction rate and selectivity towards HPA. Besides, the process is not really attractive in respect of catalyst recycle.

It would be desirable to produce the hydroformylation product at improved rate. It is therefore an object of the invention to provide a process for preparing 1,3-alkanediols and 3-hydroxyaldehydes at improved rate. It is a further object of the invention to provide a process in which essentially all of the catalyst can be conveniently recycled.

Accordingly, the invention provides a process for preparing 1,3-alkanediols and 3-hydroxyaldehydes by hydroformylating an oxirane with carbon monoxide and hydrogen in the presence of a cobalt-based catalyst and a promoter, characterized in that the cobalt-based catalyst is a non-phosphine ligated catalyst and the promoter is a lipophilic quaternary salt of a Group V element.

The oxirane comprises an organic compound, two carbon atoms of which are connected by an oxy linkage as well as by a carbon-carbon single bond. In general terms, the oxiranes comprise hydrocarbyl-epoxides, having at least 2, preferably up to 30, more preferably up to 20, most preferably up to 10 carbon atoms. The hydrocarbyl group may be aryl, alkyl, alkenyl, aralkyl, cycloalkyl, or even alkylene; straight chain or branched chain. Suitable examples of oxiranes include 1,2-epoxy(cyclo)alkanes, such as ethylene oxide, propylene oxide, 1,2-epoxyoctane, 1,2-epoxycyclohexane, 1,2-epoxy-2,4,4-trimethylhexane, and 1,2-epoxyalkenes such as 1,2-epoxy-4-pentene. Ethylene oxide and propylene oxide are preferred. In view of the demand for PDO, ethylene oxide (EO) is the oxirane most preferably used in the process of the invention.

The hydroformylation reaction is preferably carried out in a liquid solvent inert to the reactants and products, i.e., that is not consumed during the reaction. Upon completion of the reaction, the liquid solvent facilitates the separation of the hydroformylation product. The separation may be carried out by allowing the product to form a separate layer, as is disclosed in US-A-3 687 981. However, as discussed below, it is preferred to carried out the separation by extraction with an aqueous liquid. In general, ideal solvents for the hydroformylation process will (a) exhibit low to moderate polarity such that the 3-hydroxyaldehyde will be dissolved to a concentration of at least about 5 wt% under hydroformylation conditions, while significant solvent will remain as a separate phase upon extraction with the aqueous liquid, (b) dissolve carbon monoxide, and (c) be essentially non-water-miscible. By "essentially non-water-miscible" is meant that the solvent has a solubility in water at 25 °C of less than 25 wt% so as to form a separate hydrocarbon-rich phase upon extraction of the 3-hydroxyaldehyde from the hydroformylation reaction mixture. Preferably, this solubility is less than 10 wt%, most preferably less than 5 wt%. The solubility of carbon monoxide in the selected solvent will generally be greater than 0.15 v/v (1 atm, 25 °C), preferably greater than 0.25 v/v, expressed in terms of Ostwald coefficients.

The preferred class of solvents are alcohols and ethers which can be described according to the formula (1)

R¹-O-R² (1)

in which R¹ is hydrogen or a linear, branched, cyclic or aromatic C₁₋₂₀ hydrocarbyl or mono- or polyalkylene oxide and R² is a linear, branched, cylic or aromatic C₁₋₂₀ hydrocarbyl, alkoxy or mono- or polyalkylene oxide, or R¹, R² and O together form a cyclic ether. The most preferred hydroformylation solvents can be described by the formula (2) 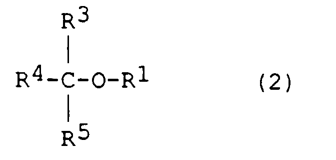 in which R¹ is hydrogen or C₁₋₈ hydrocarbyl and R³, R⁴ and R⁵ are independently selected from C₁₋₈ hydrocarbyl, alkoxy and mono- or polyalkylene oxide. Such ethers include, for example, tetrahydrofuran, methyl-t-butyl ether, ethyl-t-butyl ether, phenylisobutyl ether, ethoxyethyl ether, diethyl ether, diphenyl ether, and diisopropyl ether. Blends of solvents such as t-butylalcohol/hexane, tetrahydrofuran/toluene and tetrahydrofuran/heptane can also be used to achieve the desired solvent properties. The currently preferred solvent, because of the high yields of HPA which can be achieved under moderate reaction conditions, is methyl-t-butyl ether.

The cobalt-based catalyst may be a modified cobalt carbonyl compound, other than a phosphine-ligated compound, or an unmodified cobalt carbonyl compound. The unmodified cobalt carbonyl compounds are preferred.

The cobalt-based catalyst can be supplied to the hydroformylation reactor as a cobalt carbonyl such as dicobaltoctacarbonyl or cobalt hydridocarbonyl. It may also be supplied in essentially any other form including metal, supported metal, Raney-cobalt, hydroxide, oxide, carbonate, sulfate, acetylacetonate, salt of a fatty acid, or aqueous cobalt salt solution. If not supplied as cobalt carbonyl, operating conditions should be adjusted such that cobalt carbonyls are formed, for instance via reaction with H₂ and CO as described in J. Falbe, "Carbon Monoxide in Organic Synthesis," Springer-Verlag, NY (1970). Typically, these conditions will include a temperature of at least 50 °C and a carbon monoxide partial pressure of at least 0.8 MPa (100 psig). For more rapid reaction, temperatures of 120 to 200 °C should be employed, at CO pressures of at least 3.5 MPa (500 psig). Addition of high surface area activated carbons or zeolites, especially those containing or supporting platinum or palladium metal, is known to accelerate the formation of cobalt carbonyls.

The catalyst is preferably maintained under a stabilising atmosphere of carbon monoxide, which also provides protection against exposure to oxygen. The most economical and preferred catalyst activation and reactivation (of recycled catalyst) method involves converting the cobalt salt (or derivative) under H₂/CO in the presence of the catalyst promoter employed for hydroformylation. The conversion of Co²⁺ to the desired cobalt carbonyl is carried out at a temperature within the range of 75 to 200 °C, preferably 100 to 140 °C and a pressure within the range of 7.0 to 34.6 MPa (1000 to 5000 psig) for a time preferably less than about 3 hours. The preforming step can be carried out in a pressurised preforming reactor or in-situ in the hydroformylation reactor.

The amount of cobalt present in the reaction mixture will vary depending upon the other reaction conditions, but will generally fall within the range of 0.01 wt% to 1 wt%, preferably 0.05 to 0.3 wt%, based on the weight of the reaction mixture.

The hydroformylation reaction is carried out in the presence of a lipophilic quaternary salt of a Group V element to accelerate the reaction rate without imparting hydrophilicity (water solubility) to the active catalyst. By "lipophilic" is meant that the promoter tends to remain in the organic phase after extraction of the 3-hydroxyaldehyde with the aqueous liquid.

Lipophilic quaternary salts of Group V elements include those represented by formula (3) 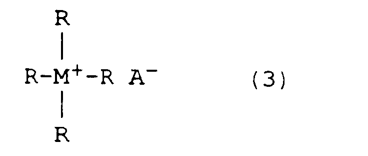 in which each R group is selected independently from unsubstituted and inertly-substituted C₁₋₂₅ linear, branched, cyclic or aromatic hydrocarbyl, alkoxy, or mono- or polyalkylene oxide, M is a Group V atom and is preferably a nitrogen, phosphorus or arsenic atom and A is a basic anion preferably having a conjugate acid of pKa >2 such as carboxylate, phenate and hydroxide, for example. Two or more of the R groups together may form a cyclic or aromatic structure. Such quaternary salts include benzyltri(n-butyl)ammonium acetate, benzyltrimethylammonium methoxide, benzyltrimethylammonium hydroxide and ethoxylated quarternary ammonium salts such as those available under the trademark "ETHOQUAD"; tetra(n-butyl)phosphonium acetate, tetraoctylphosphonium acetate, tetraphenylphosphonium hydroxide and benzyltrimethylphosphonium acetate; and tetraphenylarsonium acetate, tetra(n-butyl)arsonium acetate, and tetraoctylarsonium acetate.

The quaternary salt will be present in an amount effective to promote the hydroformylation reaction to the 3-hydroxyaldehyde, generally an amount within the range of 0.01 to 0.6 moles per mole of cobalt. An amount within the range of 0.05 to about 0.3 moles per mole of cobalt is preferred.

It is generally preferred to regulate the concentration of water in the hydroformylation reaction mixture, as excessive amounts of water reduce the selectivity towards the 1,3-alkanediols and 3-hydroxyaldehydes below acceptable levels and may induce formation of a second liquid phase. At low concentrations, water can assist in promoting the formation of the desired cobalt carbonyl catalyst species. Acceptable water levels will depend upon the solvent used, with more polar solvents generally more tolerant of higher water concentrations. For example, optimum water levels for hydroformylation in methyl-t-butyl ether solvent are believed to be within the range of 1 to 2.5 wt%.

The hydrogen and carbon monoxide will generally be introduced into the reaction vessel in a molar ratio within the range of 1:2 to 8:1, preferably 1:1.5 to 5:1.

The reaction is carried out under conditions effective to produce a hydroformylation reaction mixture comprising a major portion of the 3-hydroxyaldehyde and a minor portion of by-product. Moreover, the level of 3-hydroxyaldehyde in the reaction mixture is preferably maintained at less than 15 wt%, preferably 5 to 10 wt%. (To provide for solvents having different densities, the concentration of 3-hydroxyaldehyde in the reaction mixture can be expressed in molarity, i.e., less than 1.5M, preferably within the range of 0.5 to 1M.) Generally, the hydroformylation reaction is carried out at elevated temperature less than 100 °C, preferably 60 to 90 °C, most preferably 75 to 85 °C, and at a pressure within the range of 3.5 to 34.6 MPa (500 to 5000 psig), preferably (for process economics) 7.0 to 24.2 MPa (1000 to 3500 psig), with higher pressures generally imparting greater selectivity. The concentration of 3-hydroxyaldehyde in the intermediate product mixture can be controlled by regulation of process conditions such as oxirane concentration, catalyst concentration, reaction temperature and residence time. In general, relatively low reaction temperatures (below 100 °C) and relatively short residence times within the range of 20 minutes to 1 hour are preferred.

In the practice of the invention method, it is possible to achieve 3-hydroxyaldehyde yields (based on the oxirane conversion) of greater than 80%. For instance, with the hydroformylation of EO, formation of more than 7 wt% HPA in the dilute hydroformylation product mixture, at rates greater than 30 h⁻¹ are achievable. (Catalytic rates are referred to herein in terms of "turnover frequency" or "TOF" and are expressed in units of moles per mole of cobalt per hour, or h⁻¹.) Reported rates are based on the observation that, before a majority of the oxirane, here EO, is converted, the reaction is essentially zero-order in EO concentration and proportional to cobalt concentration.

As mentioned above, separation of the hydroformylation product mixture is carried out economically most attractively by extraction with an aqueous liquid.

Preferably the aqueous liquid is water. The amount of water added to the hydroformylation reaction product mixture will generally be such as to provide a weight ratio of water:mixture within the range of 1:1 to 1:20, preferably 1:5 to 1:15. The addition of water at this stage of the reaction may have the additional advantage of suppressing formation of undesirable heavy ends.

Extraction with a relatively small amount of water provides an aqueous phase which is greater than 20 wt% 3-hydroxyaldehyde, preferably greater than 35 wt% 3-hydroxyaldehyde, permitting economical hydrogenation of the 3-hydroxyaldehyde to the 1,3-alkanediol. The water extraction is preferably carried out at a temperature within the range of 25 to 55 °C, with higher temperatures avoided to minimise condensation products (heavy ends) and catalyst disproportionation to inactive, watersoluble cobalt compounds. In order to maximise catalyst recovery discussed supra, it is preferred to perform the water extraction under 0.5 to 1.5 MPa (50 to 200 psig) of carbon monoxide at 25 to 55 °C.

After separation, the hydroformylation product is suitably hydrogenated to yield the 1,3-alkanediol by reaction with hydrogen in the presence of a hydrogenation catalyst. Hydrogenation can be carried out in aqueous solution at an elevated temperature during at least a portion of the hydrogenation step of at least 40 °C, generally within the range of 50 to 175 °C, under a hydrogen pressure of at least 0.8 MPa (100 psig), generally within the range of 1.5 to 13.9 MPa (200 to 2000 psig). The reaction is carried out in the presence of a hydrogenation catalyst such as any of those based upon Group VIII metals including nickel, cobalt, ruthenium, platinum and palladium, as well as copper, zinc and chromium and mixtures and alloys thereof. Nickel-based catalysts, including bulk, supported and fixed-bed forms, provide acceptable activities and selectivities at moderate costs. Highest yields are achieved under slightly acidic reaction conditions.

Commercial operation will preferably include efficient cobalt catalyst recovery with essentially complete recycle of cobalt to the hydroformylation reaction. The preferred catalyst recovery process involves two steps, beginning with the above-described extraction of the 3-hydroxyaldehyde from the hydroformylation product mixture. A majority of the cobalt catalyst will remain in the organic phase, with the remaining cobalt catalyst passing into the aqueous liquid phase. This organic phase can be recycled to the hydroformylation reactor, with optional purge of heavy ends. Optional further decobalting of catalyst in the aqueous liquid phase can be effected by suitable method such as complete or partial oxidation of cobalt followed by precipitation and filtration, distillation, deposition on a solid support, or extraction using a suitable extractant.

The process enables the production of PDO in high yield and selectivity without the use of a phosphine-ligated cobalt catalyst in the hydroformylation step. The process also enables the recovery and recycle of essentially all the cobalt catalyst.

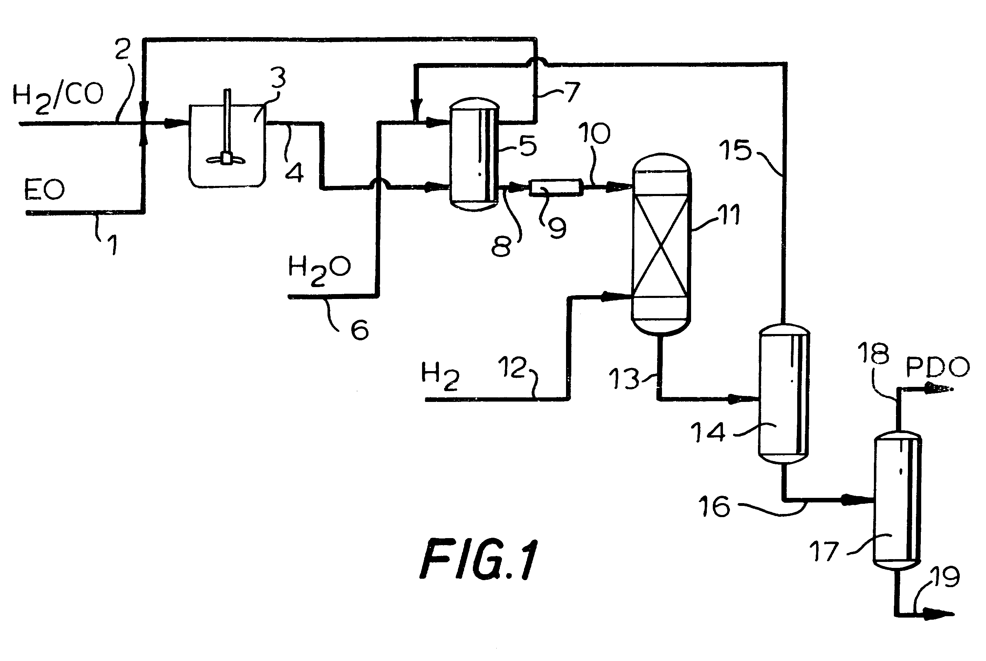

The invention process can be conveniently described by reference to Figure 1. By way of example, the hydroformylation of EO as oxirane will be described. Separate or combined streams of EO (*1*), carbon monoxide and hydrogen (*2*) are charged to hydroformylation vessel (*3*), which can be a pressure reaction vessel such as a bubble column or agitated tank, operated batch-wise or in a continuous manner. The feed streams are contacted in the presence of an unmodified cobalt-based catalyst, i.e., a cobalt carbonyl compound which has not been prereacted with a phosphine ligand.

Following the hydroformylation reaction, the hydroformylation reaction product mixture (*4*) containing HPA, the reaction solvent, PDO, the cobalt catalyst and a minor amount of reaction by-products, is passed to extraction vessel (*5*), to which an aqueous liquid, generally water and optionally a miscible solvent, are added via (*6*) for extraction and concentration of the HPA for the subsequent hydrogenation step. Liquid extraction can be effected by any suitable means, such as mixer-setters, packed or trayed extraction columns or rotating disk contactors. Extraction can if desired be carried out in multiple stages. The water-containing hydroformylation reaction product mixture can be passed to a settling tank (not shown) for resolution into aqueous and organic phases.

The organic phase containing the reaction solvent and the major portion of the cobalt catalyst can be recycled from the extraction vessel to the hydroformylation reaction via (*7*). Aqueous extract (*8*) is optionally passed through one or more acid ion exchange resin beds (*9*) for removal of any cobalt catalyst present, and the decobalted aqueous product mixture *(10)* is passed to hydrogenation vessel (*11*) and reacted with hydrogen (*12*) in the presence of a hydrogenation catalyst to produce a hydrogenation product mixture (*13*) containing PDO. The hydrogenation step may also revert some heavy ends to PDO. The solvent and extractant water *(15)* can be recovered by distillation in column (*14*) and recycled to the water extraction process via a further distillation (not shown) for separation and purge of light ends. PDO-containing stream (*16*) can be passed to one or more distillation columns (*17*) for recovery of PDO (*18*) from heavy ends (*19*).

The invention process permits the selective and economical synthesis of PDO at moderate temperatures and pressures without the use of a phosphine ligand for the hydroformylation catalyst. The process involves preparation of a reaction product mixture dilute in HPA, then concentration of this HPA by water extraction for subsequent hydrogenation of HPA to PDO.

### Comparative Example 1

0.87g of dicobaltoctacarbonyl, 1.5g toluene (internal marker), 2.0g deionised water and 146g methyl-t-butyl ether (MTBE) containing 0.2 wt% water were preheated for one hour in a 300 ml bolted-head stirred reactor at 80 °C under 4.2 MPa (600 psig) H₂ and 6.3 MPa (900 psig) 1:1 CO/H₂ synthesis gas. 10g EO were added to initiate the hydroformylation reaction. Samples were withdrawn for periodic analysis by capillary gas chromatography (with flame ionisation detector) to measure HPA hydroformylation reaction product. Sampling after about 40% conversion of EO indicated formation of 3.3 wt% HPA, at a rate (TOF) of 18 h⁻¹. This result is shown in Table 1 as run 1.

### Example 1

This experiment illustrates the use of a quaternary phosphonium salt as a promoter of cobalt-catalysed hydroformylation of EO. The experiment was repeated with addition of various amounts (based on cobalt) of tetra(n-butyl)phosphonium acetate promoter. Results are shown in Table 1. For intermediate amounts of promoter, hydroformylation was accelerated, with highest rates for M+/Co molar ratios of 0.05 to about 0.3.

**Table 1**

| Run | M+/Co | H₂O (wt%) | HPA (wt%) | TOF (h⁻¹) |
|---|---|---|---|---|
| 1 | 0.00 | 1.5 | 3.3 | 18 |
| 2 | 0.01 | 1.5 | 3.3 | 25 |
| 3 | 0.03 | 1.5 | 2.6 | 20 |
| 4 | 0.07 | 1.5 | 5.7 | 40 |
| 5 | 0.21 | 0.2 | 4.3 | 44 |
| 6 | 0.24 | 2.0 | 5.0 | 34 |
| 7 | 0.37 | 1.5 | 4.3 | 33 |
| 8 | 0.59 | 1.5 | 4.2 | 16 |

### Example 2

This example illustrates water extraction of cobalt catalyst under a carbon monoxide atmosphere in the presence of a lipophilic phosphonium acetate promoter. The reaction mixture from Run 6 of Example 1 was extracted with 30g of deionised water under 1.5 MPa (200 psig) carbon monoxide at 25 °C. 94.83g of upper organic layer containing 2184 ppmw cobalt were isolated. 24.90g of lower aqueous extract (containing most of the HPA product) were recovered and were found to contain 88 ppmw cobalt. These results correspond to 98.5% of total cobalt separated from aqueous product and remaining with the organic layer after extraction.

### Example 3

This experiment illustrates recycle of cobalt catalyst in lipophilic phosphonium acetate-promoted hydroformylation. 10.875g of dicobaltoctacarbonyl, 7g of 70% tetra(n-butyl)phosphonium acetate by weight in methanol, 18.75g toluene (marker), 25g deionised water and 1838.0g MTBE were charged to a 3.8 1 (1-gallon) bolted-head stirred reactor and heated under 13.9 MPa (2000 psig) H₂ and 5.6 MPa (800 psig) CO. After 1 hour, 123g of EO were added to initiate reaction. 1:1 CO/H₂ synthesis gas was added to replenish that depleted as hydroformylation progressed. The reaction was terminated after 50 minutes, with formation of HPA product at a rate of 39 h⁻¹. 375g of deionised water were added to extract HPA at 38 °C under 1.5 MPa (200 psig) 1:1 synthesis gas. 495g of aqueous extract were recovered, yielding HPA product corresponding to 66% of EO consumed during reaction. Less than 7% of the cobalt catalyst was extracted into the aqueous phase.

The aqueous extract was removed from the reaction, and for subsequent cycles the remaining organic phase was reheated under synthesis gas (2.6 H₂/CO) for one hour, and 120-130g of EO were added to perform recycle reactions, through 12 reaction cycles. Results are shown in Table 2.

HPA product in water extracts, as shown by gas chromatographic analysis, corresponded to yields (based upon EO consumed) of 70-89% at zero-order hydroformylation rates of 30-45 h⁻¹. Hydroformylation rates remained essentially constant for subsequent recycles, relative to the observed variability in rate measurements. Cobalt recycle (% total cobalt retained in organic phase after water extraction) in the organic phase ranged from 93 to 99%. Less than 17 ppmw phosphorus as the tetra(n-butyl)phosphonium promoter partitioned into the aqueous phase.

These experiments demonstrate the convenience of recycle of the cobalt catalyst with the organic layer following water extraction for separation from HPA product, in the presence of the lipophilic phosphonium acetate promoter. The recycle catalyst largely retains its catalytic activity and selectivity. Only minor amounts of promoter are lost to the aqueous phase upon extraction.

**Table 2**

| Cycle # | TOF (h⁻¹) | Min. HPA % Yield | Co Recycle (%) | P Loss (ppm) |
|---|---|---|---|---|
| 1 | 38.78 | 66.15 | 93.2 | 13 |
| 2 | 32.90 | 77.86 | 97.9 | NA |
| 3 | 31.80 | 74.43 | 98.7 | <15 |
| 4 | 35.70 | 80.01 | 99.5 | <15 |
| 5 | 32.94 | 78.76 | 99.5 | 16 |
| 6 | 32.21 | 76.16 | 98.0 | <15 |
| 7 | 31.93 | 71.14 | 98.7 | <15 |
| 8 | 38.33 | 78.57 | 96.6 | <15 |
| 9 | 36.48 | 76.50 | 96.9 | <15 |
| 10 | 36.49 | 72.03 | 94.6 | <15 |
| 11 | 37.44 | 74.98 | 97.3 | 14 |
| 12 | 42.66 | 89.02 | 96.3 | 10 |

### Comparative Example 2

A hydroformylation reaction was carried out with 0.87 g dicobaltoctacarbonyl, 1.5g toluene (internal marker) and 147g MTBE. After pre-equilibration of the reaction mixture for 1 hour under 1:1 CO/H₂ at 80 °C, 20g EO were added. After three hours, 3.7 wt% HPA was formed, for a rate (TOF) of 4.6 h⁻¹.

### Comparative Example 3

Comparative Example 2 was repeated with 0.077g of potassium chloride. Only 3.7 wt% HPA was formed in 3.5 hours at 80 °C for a rate (TOF) of 4.0 h⁻¹. A second run was conducted under otherwise identical conditions, with 0.06g sodium chloride instead of potassium chloride. At approximately 25% conversion of EO, a zero-order rate of only 4.3 h⁻¹ was achieved. These experiments demonstrate the limited effectiveness of neutral salts of strong acids and bases in promoting the hydroformylation reaction despite claims to the contrary in US-A-3 687 981.

### Example 4

Comparative Example 2 was repeated with the addition of 0.46g of a 40% solution of benzyltrimethylammonium methoxide in methanol. 4.73 wt% HPA was formed in less than 2 hours, for a rate (TOF) of 11.8 h⁻¹, i.e., a 2.6-fold rate increase over unpromoted Comparative Example 2.

### Example 5

Comparative Example 2 was repeated using ammonium hydroxide (Run 2, comparative),"ETHOQUAD" 2C/11 (trademark, a bis(C₁₂₋₁₃ alkyl) (hydroxyethyl)methylammonium acetate used in Runs 3 and 5), benzyltrimethylammonium methoxide (Run 4) and benzyltrimethylammonium hydroxide (Run 6* at H₂:CO = 2.3:1). Results are shown and compared with previous examples in Table 3. The quaternary ammonium acetate promoted the reaction similarly to the methoxide. While quaternary ammonium hydroxide promoted the reaction, ammonium hydroxide did not.

**Table 3**

| Run | M+ | A- | M+/Co | TOF (h⁻¹) | HPA (wt%) |
|---|---|---|---|---|---|
| 1 | - | - | 0 | 4.6 | 3.7 |
| 2 | NH₄ | OH | 0.2 | 3.6 | 3.1 |
| 3 | NR₄ | OAc | 0.1 | 12.5 | 5.4 |
| 4 | NR₄ | OMe | 0.2 | 11.9 | 4.7 |
| 5 | NR₄ | OAc | 0.5 | 12.2 | 5.6 |
| 6* | NR₄ | OH | 0.3 | 34.5 | 7.5 |

### Example 6

A series of experiments was done to determine the effect of extraction under carbon monoxide on the recovery of cobalt following hydroformylation.

Hydroformylations were conducted on 300 ml or 3.79 l (1 gallon) scales in the presence "ETHOQUAD" 2 C/11, (a trademark) as lipophilic promoter (0.1 moles, relative to cobalt) at 80 °C in MTBE, and 2.3:1 to 3.0:1 H₂/CO (with total pressures given in Table 4). Reactions were restricted to formation of less than 10 wt% HPA prior to water extraction. Water extractions were carried out at 25 to 40 °C and 0.5 to 2.2 MPa (50 to 300 psig) CO, with varying amounts of water added to give organic/aqueous phase ratios of 1.5:1 to 4:1. As can be seen from Table 2, the use of a lipophilic ammonium salt promoter and extraction under CO enabled the recycle of 90% or more of the cobalt catalyst with the hydroformylation reaction solvent, while HPA was preferentially concentrated and extracted into the aqueous phase at a greater than 10:1 ratio. Thus, cobalt catalyst and HPA were efficiently separated. Moveover, Run 7 represented a recycle of catalyst from Run 6 (3.79 1 scale). For Run 7, a hydroformylation rate of 33 h⁻¹ was obtained, compared with a rate of 35 h⁻¹ in Run 6. This illustrates that the invention enables the majority of the catalyst to be recycled in essentially active form.

**Table 4**

| | Hydrof. | | % Co* | HPA wt% | HPA wt% |
|---|---|---|---|---|---|
| Run | MPa | (psig) | | AL | OL |
| 1 | 10.4 | (1500) | 91.4 | 12.1 | 1.1 |
| 2 | 10.4 | (1500) | 91.5 | 20.3 | 0.9 |
| 3 | 10.4 | (1500) | 90.4 | 21.7 | 1.2 |
| 4 | 10.4 | (1500) | 94.4 | 23.2 | 0.8 |
| 5# | 10.4 | (1500) | 81.5 | 17.7 | 1.4 |
| 6 | 19.1 | (2750) | 99.2 | 22.7 | 0.7 |
| 7# | 19.1 | (2750) | 92.1 | 24.8 | 1.6 |

| | | | | | |
|---|---|---|---|---|---|
| * Percent total cobalt retained in organic layer. | | | | | |
| # Recycled catalyst. | | | | | |

### Comparative Example 4

Comparative Example 1 was repeated in the absence of added water and with addition of 0.14g of sodium acetate trihydrate as promoter, added at a ratio Na/Co of 0.2. HPA was formed at a rate of 41 h⁻¹. After cooling and addition of 30g deionised water for extraction, only 77% of the cobalt catalyst remained with the upper solvent layer. 23% of the cobalt was extracted with the aqueous product. This fraction corresponds approximately to the amount of sodium acetate added to promote the reaction.

### Example 7

Comparative Example 1 was repeated with addition of 0.8g of tetraoctylphosphonium acetate promoter, for a ratio of 0.29 moles promoter per mole of cobalt. At approximately 40% conversion, 3.19 wt% HPA had been formed at a rate of 27.9 h⁻¹, or a 50% rate increase over that observed in the absence of promoter in Comparative Example 1, Run 1. Ultimately, 8.1 wt% HPA was formed at 90% conversion.

Following the reaction, the reaction product mixture was cooled to room temperature. 30.5g of deionised water were added for extraction of HPA under 4.2 MPa (600 psig) synthesis gas. After 30 minutes, mixing was terminated and 33.3g of an aqueous product layer containing 27 wt% HPA were isolated. The aqueous layer contained 57 ppm cobalt, or only 1% of the total cobalt charged. The upper organic layer (110.5g) was analysed to contain 0.19 wt% cobalt, or negligible loss relative to the initial cobalt charge. Recycle of 99% of the cobalt catalyst with the organic layer represents reduction in cobalt loss by a factor of 23, relative to that observed with sodium acetate promotion in Comparative Example 4.

### Example 8

Comparative Example 1 was repeated with addition of 0.4g of tetraphenylarsonium acetate promoter, for a ratio of 0.18 moles of promoter per mole of cobalt. At about 40% conversion, 3.9 wt% HPA had been formed at a rate of 43.5 h⁻¹, or more than 2.3 times the rate observed in the absence of promoter in Comparative Example 1. The reaction was terminated following formation of 7.3 wt% HPA, at an EO conversion of approximately 80%.

Following the reaction, the mixture was cooled to room temperature. 30.2g of deionised water were added for extraction of product under 1.5 MPa (200 psig) synthesis gas. After 30 minutes, mixing was terminated and 33.0g of an aqueous product layer containing 20.5 wt% HPA were isolated. The aqueous layer contained only 39 ppm cobalt, or less than 1% of the total cobalt charged. The upper solvent layer (108.4g) was analysed to contain 0.2 wt% cobalt. Recycle of more than 99% of the cobalt catalyst with the upper solvent layer represents reduction in cobalt loss by a factor of 23, relative to that observed with sodium acetate promotion in Comparative Example 4.

## Claims

1. A process for preparing 1,3-alkanediols and 3-hydroxyaldehydes by hydroformylating an oxirane with carbon monoxide and hydrogen in the presence of a cobalt-based catalyst and a promoter, characterized in that the cobalt-based catalyst is a non-phosphine ligated catalyst and the promoter is a lipophilic quaternary salt of a Group V element.

2. A process as claimed in claim 1, wherein the oxirane is a hydrocarbyl-epoxide having of 2 up to 30 carbon atoms.

3. A process as claimed in claim 1, wherein the oxirane is ethylene oxide.

4. A process as claimed in any one of claims 1 to 3, carried out in the presence of an inert, essentially non-water-miscible solvent.

5. A process as claimed in any one of claims 1 to 4, wherein the amount of cobalt-based catalyst is in the range of 0.01 wt% to 1.0 wt% based on the weight of the reaction mixture.

6. A process as claimed in any one of claims 1 to 5, wherein the lipophilic quaternary salt of the Group V cation is represented by formula (3) 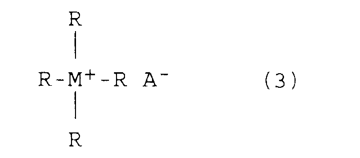 in which each R group is selected independently from unsubstituted and inertly-substituted C₁₋₂₅ linear, branched, cyclic or aromatic hydrocarbyl, alkoxy, or mono- or polyalkylene oxide, M is a Group V atom and is preferably a nitrogen, phosphorus or arsenic atom and A is a basic anion having a conjugate acid of pKa >2.

7. A process as claimed in claim 6, wherein the lipophilic quaternary salt is selected from benzyltri(n-butyl)ammonium acetate, benzyltrimethylammonium methoxide, benzyltrimethylammonium hydroxide and ethoxylated quaternary ammonium salts; tetra(n-butyl)phosphonium acetate, tetraoctylphosphonium acetate, tetraphenylphosphonium hydroxide and benzyltrimethylphosphonium acetate; and tetraphenylarsonium acetate, tetra(n-butyl)arsonium acetate, and tetraoctylarsonium acetate.

8. A process as claimed in any one of claims 1 to 7, wherein the lipophilic quaternary salt of the Group V element is present in an amount in the range of 0.01 to 0.6 moles per mole of cobalt.

9. A process as claimed in any one of claims 1 to 8, wherein the 3-hydroxyaldehyde is hydrogenated to yield the 1,3-alkanediol.

## Patentansprüche

1. Verfahren zur Herstellung von 1,3-Alkandiolen und 3-Hydroxyaldehyden durch Hydroformylieren eines Oxirans mit Kohlenmonoxid und Wasserstoff in Anwesenheit eines Katalysators auf Kobaltbasis und eines Promotors, dadurch gekennzeichnet, daß der Katalysator auf Kobaltbasis ein nicht-Phosphin-ligierter Katalysator ist und der Promotor ein lipophiles quaternäres Salz eines Gruppe V-Elements ist.

2. Verfahren nach Anspruch 1, worin das Oxiran ein Hydrocarbylepoxid mit 2 bis zu 30 Kohlenstoffatomen ist.

3. Verfahren nach Anspruch 1, worin das Oxiran Ethylenoxid ist.

4. Verfahren nach einem der Ansprüche 1 bis 3, das in Anwesenheit eines inerten, im wesentlichen mit Wasser nicht mischbaren Lösungsmittels ausgeführt wird.

5. Verfahren nach einem der Ansprüche 1 bis 4, worin die Menge des Katalysators auf Kobaltbasis im Bereich von 0,1 bis 1,0 Gew.-%, bezogen auf das Gewicht des Reaktionsgemisches, liegt.

6. Verfahren nach einem der Ansprüche 1 bis 5, worin das lipophile quaternäre Salz des Gruppe V-Kations durch die Formel (3) 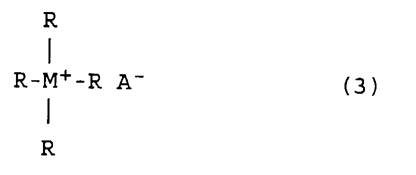 dargestellt wird, worin jede Gruppe R unabhängig unter unsubstituiertem und inert-substituiertem C₁₋₂₅ linearem, verzweigtem, cyclischem oder aromatischem Hydrocarbyl, Alkoxy oder Mono- oder Polyalkylenoxid ausgewählt ist, M ein Gruppe V-Atom ist und vorzugsweise ein Stickstoff-, Phosphor- oder Arsenatom ist und A ein basisches Anion mit einer konjugierten Säure mit einem pKa-Wert von >2 ist.

7. Verfahren nach Anspruch 6, worin das lipophile quaternäre Salz unter Benzyltri(n-butyl) ammoniumacetat, Benzyltrimethylammoniummethoxid, Benzyltrimethylammoniumhydroxid und ethoxylierten quaternären Ammoniumsalzen; Tetra(n-butyl)phosphoniumacetat, Tetraoctylphosphoniumacetat, Tetraphenylphosphoniumhydroxid und Benzyltrimethylphosphoniumacetat; und Tetraphenylarsoniumacetat, Tetra(n-butyl)arsoniumacetat und Tetraoctylarsoniumacetat ausgewählt ist.

8. Verfahren nach einem der Ansprüche 1 bis 7, worin das lipophile quaternäre Salz des Gruppe V-Elements in einer Menge im Bereich von 0,01 bis 0,6 Mol je Mol Kobalt zugegen ist.

9. Verfahren nach einem der Ansprüche 1 bis 8, worin der 3-Hydroxyaldehyd zur Ausbildung des 1,3-Alkandiols hydriert wird.

## Revendications

1. Procédé de préparation de 1,3-alcanediols et de 3-hydroxyaldéhydes par l'hydroformylation d'un oxiranne avec le monoxyde de carbone et l'hydrogène en présence d'un catalyseur à base de cobalt et d'un promoteur, caractérisé en ce que le catalyseur à base de cobalt est un catalyseur lié à un composé qui n'est pas une phosphine et le promoteur est un sel quatemaire lipophile d'un élément du groupe V.

2. Procédé suivant la revendication 1, caractérisé en ce que l'oxiranne est un hydrocarbyl-époxyde possédant de 2 jusqu'à 30 atomes de carbone.

3. Procédé suivant la revendication 1, caractérisé en ce que l'oxiranne est l'oxyde d'éthylène.

4. Procédé suivant l'une quelconque des revendications 1 à 3, caractérisé en ce qu'on l'entreprend en présence d'un solvant essentiellement non miscible à l'eau et inerte.

5. Procédé suivant l'une quelconque des revendications 1 à 4, caractérisé en ce que la quantité de catalyseur à base de cobalt fluctue dans la plage de 0,01% en poids à 1,0% en poids sur base du poids du mélange réactionnel.

6. Procédé suivant l'une quelconque des revendications 1 à 5, caractérisé en ce que le sel quaternaire lipophile du cation du groupe V est représenté par la formule (3) : 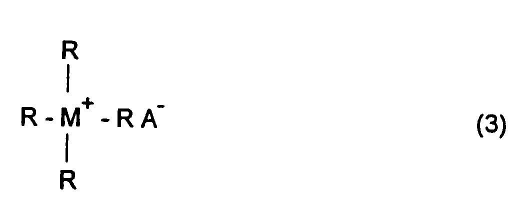 dans laquelle chaque symbole R est indépendamment choisi parmi les radicaux oxyde de mono- ou polyalkylène, alcoxy ou hydrocarbyle en C₁ à C₂₅ linéaire, ramifié, cyclique ou aromatique, à substitution inerte, M est un atome du groupe V et est, de préférence, un atome d'azote, de phosphore ou d'arsenic et A est un anion basique, de préférence, possédant un acide conjugué d'un pKa supérieur à 2.

7. Procédé suivant la revendication 6, caractérisé en ce que l'on choisit le sel quatemaire lipophile parmi l'acétate de benzyltri(n-butyl)ammonium, le méthylate de benzyltriméthylammonium, l'hydroxyde de benzyltriméthylammonium et des sels d'ammonium quaternaire éthoxylés; l'acétate de tétra(n-butyl)phosphonium, l'acétate de tétraoctylphosphonium, l'hydroxyde de tétraphénylphosphonium et l'acétate de benzyltriméthylphosponium et l'acétate de tétraphénylarsonium, l'acétate de tétra(n-butyl)arsonium et l'acétate de tétraoctylarsonium.

8. Procédé suivant l'une quelconque des revendications 1 à 7, caractérisé en ce que le sel quatemaire lipophile de l'élément du groupe V est présent en une proportion qui varie de 0,01 à 0,6 mole par mole de cobalt.

9. Procédé suivant l'une quelconque des revendications 1 à 8, caractérisé en ce que le 3-hydroxyaldéhyde est hydrogéné pour donner le 1,3-alcanediol.
